# DEMANDE DE BREVET EUROPEEN

(11) **EP 1 754 513 A2**
(43) Date de publication de la demande: **21.02.2007**
(21) Numéro de dépôt: 06291204.3
(22) Date de dépôt: 25.07.2006
(51) Int. Cl.: A61Q 5/00, A61Q 19/00, A61K 8/36, A61P 17/00

(54) **Utilisation de l'acide 8-hexadécène-1,16-dicarboxylique comme agent de soin destiné à favoriser la cohésion de la couche cornée**

(30) Priorité: 17.08.2005 FR 0552526
(71) Demandeur: L'ORÉAL, 75008 Paris (FR)
(72) Inventeur: Bernard, Dominique, 75015 Paris (FR); Mahe, Yann, 91390 Morsang sur Orge (FR)
(74) Mandataire: Bernstein, Claire Jacqueline

(57) **Abrégé**

L'invention concerne notamment l'utilisation non thérapeutique de l'acide 8-hexadécène-1,16-dicarboxylique dans ou pour la préparation d'une composition, comme agent destiné à favoriser la cohésion et/ou l'organisation de la couche cornée et ainsi notamment favoriser l'homogénéité et/ou la clarté du teint, améliorer l'homogénéité et/ou la tenue des compositions de maquillage sur la peau ou les lèvres, prévenir l'altération de la couche cornée induite par un actif cosmétique ou dermatologique, ou encore favoriser la cohésion de la couche cornée dans la préparation des épidermes reconstruits et/ou des peaux reconstruites.

## Description

Le domaine de l'invention concerne notamment l'aspect et la cohésion de la couche cornée et ses applications en particulier dans le domaine du soin et/ou du maquillage de la peau et des lèvres.

L'invention concerne notamment l'utilisation non thérapeutique de l'acide 8-hexadécène-1,16-dicarboxylique dans ou pour la préparation d'une composition, comme agent destiné à favoriser la bonne organisation et cohésion de la couche cornée et ainsi notamment favoriser l'homogénéité et/ou la clarté du teint, améliorer l'homogénéité et/ou la tenue des compositions de maquillage sur la peau ou les lèvres, prévenir l'altération de la couche cornée induite par un actif cosmétique ou dermatologique, ou encore favoriser la résistance et la cohésion de la couche cornée dans la préparation des épidermes reconstruits et/ou des peaux reconstruites.
De préférence, l'acide 8-hexadécène-1,16-dicarboxylique est présent dans la composition en une quantité allant de 0,00001 à 8%, pour ces applications, l'acide 8-hexadécène-1,16-dicarboxylique est efficace même pour des concentrations inférieures à 0,005% en poids par rapport au poids total.

L'invention porte également sur des compositions de maquillage comprenant au moins de l'acide 8-hexadécène-1,16-dicarboxylique et caractérisées par une forme particulière, ainsi que sur des compositions cosmétiques ou dermatologiques associant (a) au moins l'acide 8-hexadécène-1,16-dicarboxylique et (b) au moins un autre acide choisi parmi l'acide lactique, l'acide glycolique, et l'acide ascorbique, dans un rapport quantitatif allant de 0,005 :1 à 0,05 :1, de préférence un rapport de 0,01 :1.
L'invention porte encore sur des procédés cosmétiques visant à améliorer l'aspect de surface et/ou le confort de la peau ou des lèvres ou le teint de la peau mettant en oeuvre lesdites compositions.

La peau humaine est constituée de deux compartiments à savoir un compartiment profond, le derme, et un compartiment superficiel, l'épiderme.

Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, qui sont synthétisés par le fibroblaste. On y trouve aussi des leucocytes, des mastocytes ou encore des macrophages tissulaires. Le derme est également parcouru de vaisseaux sanguins et de fibres nerveuses.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.
L'épiderme est conventionnellement divisé en une couche basale de kératinocytes qui constitue la couche germinative de l'épiderme, une couche dite épineuse constituée de plusieurs couches de cellules polyédriques disposées sur les cellules germinatives, une couche dite granuleuse constituée de cellules aplaties contenant des inclusions cytoplasmiques distinctes, les grains de kératohyaline, et enfin une couche supérieure appelée couche cornée (ou *stratum corneum*), constituée de cornéocytes, qui sont des structures cellulaires momifiées, anucléées qui dérivent des kératinocytes.

Les cornéocytes sont principalement composés d'une matrice fibreuse contenant des cytokératines, entourée d'une structure très résistante de 15 nm d'épaisseur, appelée enveloppe cornée ou cornifiée. L'empilement de ces cornéocytes constitue la couche cornée qui est la plus superficielle et est responsable de la fonction barrière et de l'aspect de l'épiderme.

On sait que la bonne cohésion et l'organisation de la couche cornée sont en partie liées à l'élaboration de l'enveloppe cornée, contrôlée notamment par des enzymes de la famille des transglutaminases qui utilisent un ensemble de protéines « précurseurs » comme substrats. L'élasticité et la solidité de la couche cornée est établie à travers un réseau entre notamment l'enveloppe cornée, les cornéodesmosomes et les filaments intermédiaires de cytokératines.

Mais cette bonne organisation de la couche cornée de la peau ou des lèvres peut être altérée ou modifiée par des facteurs environnementaux de type agents irritants ou polluants (détergents, pollution, fumée de cigarettes...), sollicitations mécaniques (frottements, abrasion, rasage), déséquilibres thermiques ou climatiques (froid, sécheresse, radiations), xénobiotiques (micro-organismes, allergènes), traitements chimiques cosmétiques ou dermatologiques (peeling, traitement anti-acné...), ou par des facteurs physiologiques (âge, stress...).

Cette altération de la couche cornée ou rupture de la continuité de la surface de la peau ou des lèvres peut se répercuter sur l'homogénéité de leur surface, en particulier l'homogénéité et/ou la clarté du teint de la peau. Ces variabilités de cohésion et/ou de structuration de la couche cornée peuvent également modifier le microrelief ou les propriétés physico-chimiques du *stratum corneum* conduisant à une moins bonne tenue des compositions de soin et/ou de maquillage appliquées sur la peau ou les lèvres et par conséquent à des répartitions hétérogènes de maquillage pouvant générer des tâches disgracieuses.

Par ailleurs, ces variations de cohésion et/ou de structuration de la couche cornée peuvent participer au développement de désordres pathologiques, tels que :
- des hyperkératoses, caractérisées par une couche cornée épaissie et par une desquamation anormale (ex : xéroses, ichthyoses, hyperkératoses réactionnelles...);
- des leukokératoses, caractérisées par une transdifférenciation ou métaplasie, au niveau de muqueuses, malpighiennes ou non, mais normalement non cornifiées, qui deviennent cornifiées (ex : leukokératose du col utérin au cours du prolapsus, leukokératoses buccales, lésions tumorales bénignes kératosiques des muqueuses malpighiennes...) ;
- des troubles trophiques cutanés, caractérisés à l'inverse par un amincissement de l'épiderme et en particulier de la couche cornée, se traduisant par une fragilité excessive du revêtement cutané (ex : troubles trophiques cutanés des membres inférieurs chez les patients porteurs de pathologies vasculaires de type varices, artériopathies ; troubles trophiques cutanés dans le cadre d'un syndrome algodystrophique ; troubles trophiques consécutifs à une cicatrisation anormale...).

On sait aussi que la cohésion et l'organisation tridimensionnelle de la couche cornée sont des étapes importantes des procédés de préparation d'épidermes et/ou de peaux reconstruites qui signent souvent leur bonne maturation et qualité.

On comprend donc de ce qui précède le besoin de disposer de produits capables de favoriser et/ou restaurer la bonne organisation et mise en place des enveloppes cornées, garante de l'homogénéité et de la cohésion de la couche cornée à la surface de la peau et des lèvres.

Pour favoriser la cohésion la couche cornée, on peut notamment favoriser la dernière étape de sa mise en place. Par étape de mise en place de la couche cornée, on entend le processus au cours duquel les précurseurs protéiques produits par les kératinocytes sont réticulés notamment par l'action de la transglutaminase et insolubilisés, ces protéines pouvant également être insolubilisées par liaisons à certains céramides pour former des structures hydrophobes.

Or la Demanderesse vient de montrer de manière inattendue que l'acide 8-hexadécène-1,16-dicarboxylique à faible concentration non irritante pour la peau pouvait répondre à ce besoin. En effet, il a maintenant été montré que l'acide 8-hexadécène-1,16-dicarboxylique à faible concentration induit l'expression de protéines impliquées dans la cohésion de la couche cornée (ex : LEP16) ou la cohésion intercornéocytaire (cornéodesmosine).
La Demanderesse a également obtenu des résultats similaires ou complémentaires avec l'acide ascorbique, l'acide lactique et l'acide glycolique. Dans ce cas particulier, de ces composés, une concentration 10 fois supérieure à l'acide 8-hexadécène-1,16-dicarboxylique est cependant nécessaire pour obtenir une activité équivalente.
La LEP16 est un des membres de la famille des LEPs (Late Envelope Proteins) qui apparaissent tardivement au cours de la formation de l'enveloppe cornée (Marshall D et al., PNAS, 2001, vol 98, n°23, 13031-13036) ; ces protéines jouent un rôle dans l'interaction matrice-enveloppe durant la bonne formation de l'enveloppe cornée et du squame.
Les LEPs ont été récemment décrites comme nouveaux substrats des transglutaminases, connues pour être impliquées dans la formation de l'enveloppe cornée par pontage entre elles et de nombreuses protéines, dont les principales sont l'involucrine, la loricrine, l'élafine, les cystatines, les pancornulines (ou SPR : Small Proline Rich), des cytokératines, les desmoplakines I et II, des desmogléines et la cornéodesmosine.

L'utilisation de l'acide 8-hexadécène-1,16-dicarboxylique est donc avantageuse comme agent de soin destiné à favoriser et/ou restaurer la cohésion de la couche cornée et assurer ainsi une bonne homogénéité de la surface de la peau et des lèvres favorisant un meilleur rendu esthétique des compositions de maquillage (homogénéité et tenue) ; l'utilisation de cet acide est également avantageuse pour la préparation de compositions pharmaceutiques destinées au traitement de pathologies cutanées liées à une cornification anormalement faible de la couche cornée.
Elle est également avantageuse dans des procédés de préparation d'épidermes et/ou de peaux reconstruites ou pour la bonne différentiation et maturation de follicule pileux en survie.

L'acide 8-hexadécène-1,16-dicarboxylique ou acide 9-octadécène dioïque est un composé qui se trouve de façon prédominante sous forme *cis,* obtenu par biofermentation de l'acide oléique en présence d'une levure mutante de l'espèce *Candida.* Il présente notamment des propriétés blanchissantes et anti-microbiennes permettant d'envisager son utilisation dans des produits dépigmentants, déodorants, antipelliculaires et anti-acné, comme décrit par J. W. WIECHERS et al. dans Cosmetics & Toiletries, Vol. 117, n° 7, p.55-68 (Juillet 2002) et dans SÖFW Journal, 128, p.2-8 (2002).
Il a en outre été proposé dans la demande DE 10305965 de l'utiliser comme agent antioxydant dans des compositions cosmétiques pour rajeunir ou revitaliser la peau à des concentrations comprises entre 0,005 et 20% en poids.
Ce composé est aussi décrit comme agent de peeling chimique (WO 2005/089707) à des concentrations d'au moins 10%.

Mais à la connaissance de la Demanderesse, il n'a jamais été décrit ni suggéré d'utiliser l'acide 8-hexadécène-1,16-dicarboxylique comme agent de soin pour améliorer et/ou restaurer la cohésion de la couche cornée et ainsi améliorer l'homogénéité du teint, améliorer l'homogénéité et/ou la tenue des compositions de maquillage sur la peau ou les lèvres, prévenir l'altération de la couche cornée induite par un actif cosmétique ou dermatologique, comme par exemple un agent kératolytique ou anti-acné, ou encore favoriser la cohésion et la bonne organisation de la couche cornée dans la préparation d'épidermes reconstruits et/ou des peaux reconstruites.
En particulier, l'acide 8-hexadécène-1,16-dicarboxylique est présent dans la composition à faible concentration, c'est-à-dire en une quantité nécessaire pour obtenir l'effet recherché, à savoir un effet de soin destiné à améliorer et/ou restaurer la cohésion et la bonne organisation de la couche cornée. La concentration en acide 8-hexadécène-1,16-dicarboxylique sera adaptée par l'homme du métier pour obtenir de préférence une concentration en acide libre strictement inférieure à 0,005%. Ainsi, si l'acide 8-hexadécène-1,16-dicarboxylique est partiellement salifié ou estérifié, sa concentration totale dans la composition pourra être plus élevée ; il en est de même si l'acide 8-hexadécène-1,16-dicarboxylique est apporté sous une forme à libération prolongée, par exemple dans un vecteur comme des nanocapsules ou dans un patch, qui apportera progressivement la quantité efficace au site d'action.

L'invention concerne donc l'utilisation non thérapeutique de l'acide 8-hexadécène-1,16-dicarboxylique dans ou pour la préparation d'une composition, comme agent destiné à favoriser la cohésion et bonne organisation de la couche cornée.
Pour ces applications l'acide 8-hexadécène-1,16-dicarboxylique peut être présent à une concentration allant de 0,00001 à 8%, ou encore à moins de 5.10⁻³% en poids par rapport au poids total de la composition.

Plus préférentiellement, l'acide 8-hexadécène-1,16-dicarboxylique est présent dans la composition en une quantité allant de 0,0001 à 0,0005% en poids par rapport au poids total.

L'acide 8-hexadécène-1,16-dicarboxylique utilisé selon l'invention peut être sous forme cis, sous forme trans, ou sous un mélange de ces deux formes. Il est notamment disponible dans le commerce auprès de la société UNIQUEMA sous la dénomination commerciale Arlatone Dioic DCA.

L'acide 8-hexadécène-1,16-dicarboxylique présent dans la composition sera notamment destiné à :
- favoriser l'homogénéité et/ou la clarté du teint ;
- favoriser l'homogénéité et/ou la tenue d'une composition de maquillage appliquée sur la peau ou les lèvres ;
- favoriser la ré-épithélialisation et/ou la régénération de la peau ou des lèvres ; en particulier dans le cas de peaux et/ou lèvres altérées par des agressions externes (froid, frottements mécaniques...), des brûlures, une exposition UV ou des traitements peelings ;
- favoriser la ré-épithélialisation et/ou la régénération du contour de la racine pilaire, en assurant une meilleure cohésion à la jonction infundibulaire entre l'épiderme et la tige pilaire. Les compositions ou l'acide 8-hexadécène-1,16-dicarboxylique pourront ainsi favoriser la repousse et la qualité et/ou diminuer la chute du cheveu, éventuellement en association avec d'autres actifs. Ils pourront également être utilisés pour favoriser la croissance des cils ;
- favoriser la ré-épithélialisation et/ou la régénération des ongles en particulier au niveau de la cuticule. En favorisant la cornification de l'ongle, on vise en particulier à traiter ou prévenir le phénomène des ongles cassants ;
- améliorer le confort et la résistance de la peau, du cuir chevelu ou des lèvres, altéré par au moins une condition choisie parmi le froid, les rayonnements UV, ou des frottements mécaniques (ex : rasage, épilation...) ;
- préparer la peau à l'action d'un peeling ou à une exposition solaire.

L'acide 8-hexadécène-1,16-dicarboxylique est aussi avantageusement utilisé selon l'invention pour uniformiser la couleur du bronzage.

Pour toutes ces applications, l'acide 8-hexadécène-1,16-dicarboxylique est actif à faible concentration, dès 0,00001%, mais il pourra être utilisé à des concentrations supérieures, cette quantité pourra varier par exemple de 0,00001 à 8%, ou à 5% en poids par rapport au poids total de la composition, de préférence supérieure ou égale à 0,0001% ; des quantités adaptées à la mise en oeuvre de l'invention seront notamment de 0,0001 à 0,005% ou encore 0,0001 à 0,0005% en poids par rapport au poids total de la composition.
Avantageusement, cette quantité n'excédera pas 8% en poids par rapport au poids total de la composition, de préférence n'excédera pas 5% en poids par rapport au poids total de la composition.

L'invention concerne également l'utilisation non thérapeutique de l'acide 8-hexadécène-1,16-dicarboxylique dans ou pour la préparation d'une composition, comme agent destiné à prévenir une altération de la couche cornée induite par l'action d'un actif cosmétique ou dermatologique présent dans ladite composition, et/ou favoriser sa restauration.
La composition selon l'invention peut en effet être une composition cosmétique ou dermatologique et contenir au moins un actif à effet secondaire potentiellement délétère sur la résistance et la cohésion de la couche cornée. Il peut s'agir par exemple d'un actif cosmétique ou dermatologique choisi parmi le groupe des agents kératolytiques ou desquamants, des agents antiséborrhéiques et/ou anti-acnéiques, des agents comédolytiques, et leurs mélanges.

Comme exemples d'agents kératolytiques, on peut citer notamment : les α-hydroxy-acides comme les acides glycolique, lactique, dioique, malique, citrique, tartrique, mandélique, et leurs dérivés ; les β-hydroxy-acides comme l'acide salicylique et ses dérivés ; les α-céto-acides comme l'acide ascorbique ou vitamine C et ses dérivés ; les β-céto-acides ; les rétinoïdes comme le rétinol et ses esters, le rétinal, l'acide rétinoïque et ses dérivés, ceux décrits dans les documents FR-A-2 570 377, EP-A-199 636, EP-A-325 540, EP-A-402 072.
Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée et certains de ses dérivés; l'acide gentisique ; les oligofucoses ; l'acide cinnamique ; l'extrait de Saphora japonica ; le resvératrol ; les détergents et certains dérivés d'acide jasmonique ;
- et/ou sur les activités des enzymes impliquées dans la dégradation des cornéodesmosomes, telles que la stratum corneum chymotryptic enzyme (SCCE) voire d'autres protéases (trypsin-like, chymotrypsin-like, cathepsin D) ainsi que d'autres catégories d'hydrolases (ex : glycosidases, céramidases). On peut citer les agents chélatant des sels minéraux : l'EDTA; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine ; l'urée ou certains de ses dérivés.

Comme exemples d'agents antiséborrhéiques et/ou antiacnéiques, on peut citer notamment :
- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5^{®} ;
- un extrait de Laminaria saccharina commercialisé notamment par la société SECMA sous la dénomination commerciale Phlorogine^{®} ;
- un extrait de Spiraea ulmaria commercialisé notamment par la société SILAB sous la dénomination commerciale Sebonormine^{®} ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED ;
- des extraits de plantes du genre Silybum ; et
- des extraits d'Eugenia caryophyllata contenant de l'eugénol et du glucoside d'eugényle ;
- des acides sulfoniques tels que décrits dans le brevet EP 0 728 474 (L'OREAL).

Comme exemples d'agents comédolytiques, on peut citer notamment : l'acide salicylique, l'Hepes.

La présence d'acide 8-hexadécène-1,16-dicarboxylique à faible concentration dans la composition cosmétique ou dermatologique selon l'invention permettra de prévenir et/ou diminuer l'effet secondaire potentiellement délétère sur la cohésion et l'organisation de la couche cornée d'un actif cosmétique ou dermatologique présent dans ladite composition.

L'invention concerne également l'utilisation de l'acide 8-hexadécène-1,16-dicarboxylique pour la préparation d'une composition dermatologique destinée à traiter les affections cutanées liées à une formation anormale de la couche cornée, en particulier les hyperkératoses, les leukokératoses, les parakératoses et les troubles trophiques cutanés.

L'acide 8-hexadécène-1,16-dicarboxylique est présent dans la composition en une quantité nécessaire pour obtenir l'effet recherché, à savoir une amélioration et/ou une restauration de la maturation et/ou de la cohésion de la couche cornée.
Comme indiqué plus haut, la concentration d'acide 8-hexadécène-1,16-dicarboxylique sera adaptée par l'homme du métier, en fonction de la forme sous laquelle il est présent dans la composition, pour obtenir une quantité efficace. Cette quantité pourra aller de 0,00001 à 5%, de 0,00001 à 8% en poids par rapport au poids total de la composition, des quantités adaptées à la mise en oeuvre de l'invention seront notamment de 0,0001 à 0,005% ou encore 0,0001 à 0,0005% en poids par rapport au poids total de la composition.
Avantageusement, cette quantité n'excédera pas 8% en poids par rapport au poids total de la composition et de préférence n'excédera pas 5% en poids par rapport au poids total de la composition.

De façon avantageuse, l'acide 8-hexadécène-1,16-dicarboxylique est associé dans la composition de l'invention à au moins un autre acide choisi parmi l'acide lactique, l'acide glycolique, l'acide ascorbique et leurs mélanges, pour lesquels la Demanderesse a pu montrer des effets additionnels ou complémentaires sur la maturation de la couche cornée, la cohésion intercornéocytaire et/ou la ré-épithélialisation.
L'invention a donc également pour objet l'utilisation d'au moins un composé choisi parmi l'acide lactique et l'acide glycolique comme agent pour favoriser la maturation et/ou la cohésion de la couche cornée.
Lorsque l'acide 8-hexadécène-1,16-dicarboxylique et les autres agents sont utilisés en association, cela permet en outre de diminuer les quantités efficaces de chaque actif présent dans la composition.

Les compositions selon l'invention pourront également contenir de l'urée qui favorisera l'activité finale d'amélioration de la couche cornée. L'acide 8-hexadécène-1,16-dicarboxylique et au moins un autre acide et/ou l'urée, dans des quantités comprises entre 0,01 et 10% en poids, pourront être utilisés de façon simultanée, séparée, décalée et/ou séquencée dans le temps.

En particulier, l'acide 8-hexadécène-1,16-dicarboxylique et l'autre acide choisi parmi l'acide lactique, l'acide glycolique, l'acide ascorbique, chacun des ces acides pouvant être présent dans la composition dans des quantités comprises entre 0,01 et 10% en poids, et leurs mélanges sont présents dans la composition dans un rapport quantitatif allant de 0,005 : 1 à 0,05 : 1, de préférence dans un rapport de 0,01 : 1.

L'objet de la présente invention se rapporte encore à des procédés cosmétiques d'uniformisation de la couleur du bronzage comprenant l'application sur la peau de l'acide 8-hexadécène-1,16-dicarboxylique avant ou après l'exposition au soleil.
La quantité d'acide 8-hexadécène-1,16-dicarboxylique pourra aller de 0,00001 à 5%, de 0,00001 à 8% en poids par rapport au poids total de la composition, des quantités adaptées à la mise en oeuvre de l'invention seront notamment de 0,0001 à 0,005% ou encore 0,0001 à 0,0005% en poids par rapport au poids total de la composition.

Les compositions selon l'invention peuvent être à usage cosmétique ou dermatologique.
Lorsqu'il s'agira d'une composition dermatologique, il s'agira notamment d'une composition de peeling ou d'une composition anti-acné.

Préférentiellement, la composition de l'invention est à usage cosmétique destiné à améliorer l'homogénéité de surface de la peau ou des lèvres, en particulier l'homogénéité du teint. Il s'agit en particulier d'une composition de soin et/ou de maquillage de la peau
ou des lèvres, qui peut être notamment sous la forme d'une base de soin pour la peau, d'une crème de soin (crème de jour, de nuit, anti-rides), d'une base de maquillage, ou d'une crème de soin teintée, sous la forme d'un fond de teint, de consistance fluide, semi-solide ou solide.

Elle peut se présenter sous toutes les formes galéniques normalement utilisées dans les domaines cosmétique et dermatologique, adaptées à la voie orale ou topique, préférentiellement à la voie topique.

Pour une administration par voie orale, en particulier en 'cosmétique orale', elle peut se présenter notamment sous forme de capsules, de gélules, dragées, de granulés, de pâte à mâcher, de gels, de sirops buvables, de comprimés ou de toute autre forme connue de l'homme du métier.

Pour une application topique sur la peau, le cuir chevelu ou les lèvres, la composition peut avoir la forme d'une solution aqueuse, hydroalcoolique ou huileuse éventuellement gélifiée, d'émulsion de consistance liquide ou semi-liquide du type lait, obtenue par dispersion d'une phase grasse dans une phase aqueuse (H/E) ou inversement (E/H), d'une émulsion triple (E/H/E ou H/E/H), ou de suspension ou émulsion de consistance molle, semi-solide ou solide de type crème ou gel, d'un produit anhydre liquide, pâteux
ou solide ou encore de microémulsions, de microcapsules, de microparticules ou d'une dispersion vésiculaire de type ionique (liposomes ou oléosomes) et/ou non ionique (niosomes) et/ou d'une dispersion de nanosphères.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse. Elle peut également se présenter sous la forme d'un stick, d'une pâte, d'une laque à lèvres ou d'une poudre, d'un fond de teint solide ou semi-solide, d'un mascara à appliquer sur les cheveux et/ou les cils. Elle peut notamment être sous forme de patch,
ou dispositif de délivrance transdermique, destiné à une action sur le site d'application ou une action à distance par diffusion des actifs dans le compartiment cutané.

Lorsque la composition est une émulsion, la proportion de la phase grasse de la composition considérée peut aller par exemple de 5 à 80 % en poids, et notamment de 5 à 50 % en poids par rapport au poids total de la composition.

La phase aqueuse peut être constituée essentiellement d'eau ou comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

Cette phase aqueuse (eau et éventuellement le solvant organique miscible à l'eau) peut être présente dans la composition de base en une teneur allant de 1 % à 95 % en poids, notamment allant de 3 % à 80 % en poids, et en particulier allant de 5 % à 60 %, en poids par rapport au poids total de la composition de base.

La phase grasse de la composition peut contenir des composés gras ou huileux, et éventuellement des cires, des gommes, des corps gras pâteux d'origine végétale, animale, minérale ou de synthèse, siliconé ou non.

Comme huiles, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le düsostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant
ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ; et
- leurs mélanges.

Les cires peuvent être hydrocarbonées, siliconées et/ou fluorées, comportant éventuellement des fonctions ester ou hydroxyle. Elles sont notamment d'origine naturelle.
La cire peut représenter de 0,01 à 10 % en poids, notamment de 0,1 à 5 % en poids, par rapport au poids total de la composition.
Comme cire utilisable dans la composition de l'invention, on peut citer la cire d'abeilles la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméthicone ayant de 16 à 45 atomes de carbone.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.
On peut également citer les polyesters résultant de l'estérification d'un acide carboxylique et d'un ester acide hydroxycarboxylique aliphatique. Par exemple, le RISOCAST^{®} DA-L (ester issu de la réaction d'estérification de l'huile de ricin hydrogéné avec de l'acide dilinoléïque dans des proportions de 2 pour 1) et le RISOCAST^{®} DA-H (ester résultant de l'estérification de l'huile de ricin hydrogénée avec de l'acide isostéarique dans des proportions de 4 pour 3) commercialisés par la société japonaise KOKYU ALCOHOL KOGYO.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société DOW CORNING sous les noms commerciaux de DC2503^{®} et DC25514^{®} et leurs mélanges.

La composition peut contenir également les adjuvants habituels dans le domaine cosmétique, tels que les charges, les matières colorantes, les actifs cosmétiques hydrophiles ou lipophiles, les épaississants, les émulsionnants, les gélifiants hydrophiles
ou lipophiles, les tensioactifs, les hydratants, les adoucissants, les séquestrants, les parfums, les neutralisants, les conservateurs, les antioxydants, les filtres UV, les bactéricides, les oligoéléments, les absorbeurs d'odeurs, les ajusteurs de pH et leurs mélanges.
Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse ou dans la phase aqueuse.
En tout état de cause, ces adjuvants, ainsi que leurs proportions, seront choisis de manière à ne pas nuire aux propriétés recherchées selon l'invention.

Comme émulsionnants et co-émulsionnants utilisables, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H disponibles par exemple sous les dénominations commerciales ABIL WE09, ABIL EM 90 et ABIL EM97 auprès de la société Degussa Goldschmidt ou le mélange de stéarate d'éthylène glycol acétyle et de tristéarate de glycéryle commercialisé par la société Guardian sous la dénomination commerciale UNITWIX.

Comme gélifiants hydrophiles utilisables, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Par charges, il faut comprendre des particules de toute forme, incolores ou blanches, minérales ou de synthèse, insolubles dans le milieu de la composition quelle que soit la température à laquelle la composition est fabriquée.
Les charges peuvent être notamment choisies parmi les charges minérales ou organiques de toute forme, plaquettaires, sphériques ou oblongues, quelle que soit la forme cristallographique (par exemple feuillet, cubique, hexagonale, orthorombique, etc) . On peut citer le talc, le mica, la silice, le kaolin, l'amidon réticulé par l'anhydride octénylsuccinique commercialisé par la société National Starch sous la dénomination DRY FLO PLUS (28-1160) ; les particules de polyamide et notamment celles vendues sous la dénomination ORGASOL par la société Atochem ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de types polyméthacrylate (PMMA), les élastomères de silicone et les poudres de silice de type SUNSPHERES, les microbilles de résine de silicone telles que celles commercialisées sous la dénomination TOSPEARL par la société Toshiba Silicone ; et leurs mélanges.
Ces charges peuvent être présentes dans des quantités allant de 0,01 à 20 % en poids et de préférence de 1 à 10 % en poids par rapport au poids total de la composition de base.

Comme matières colorantes, on peut citer notamment les pigments monochromes, les nacres, les pigments réfléchissants qui émettent une couleur ou n'en émettent pas, les pigments interférentiels, les colorants liposolubles, les colorants hydrosolubles, et leurs mélanges.
Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les pigments peuvent avoir subi un traitement de surface.

Comme actifs cosmétiques hydrophiles ou lipophiles adaptés à une utilisation dans les compositions de l'invention, on utilisera notamment des actifs cosmétiques destinés à améliorer l'aspect de surface et/ou le confort de la peau, du cuir chevelu ou des lèvres, ou le teint de la peau.

Cet actif pourra être notamment choisi parmi un agent stimulant la synthèse de macromolécules dermiques ou épidermiques et/ou empêchant leur dégradation, un agent stimulant la prolifération des fibroblastes ou des kératinocytes et/ou la différenciation des kératinocytes, un agent hydratant, un agent dépigmentant, un agent favorisant la coloration de la peau, un agent anti-pollution ou anti-radicalaire, un agent dermo-relaxant, un agent tenseur et leurs mélanges.

### Agent stimulant la synthèse de macromolécules dermiques ou épidermiques et/ou empêchant leur dégradation

Les cellules du derme, en particulier les fibroblastes, produisent des molécules de collagène, d'élastine et de glycoprotéines. Avec l'effet de l'âge ou bien encore sous l'effet des rayons UV, il se produit une diminution notable de ces molécules ainsi qu'une dégradation des fibres de collagène et d'élastine sous l'effet de la collagénase ou de l'élastase.

Parmi les actifs stimulant les macromolécules du derme ou empêchant leur dégradation, on peut citer ceux qui agissent :
- soit sur la synthèse du collagène, tels que les extraits de Centella asiatica ; les asiaticosides et dérivés ; l'acide ascorbique ou vitamine C et ses dérivés, tels que ses sels ou ses esters, en particulier le 5,6-di-O-diméthylsilylascorbate (vendu par la Sté Exsymol sous la référence PRO-AA), le sel de potassuim du dl-alpha-tocopheryl-dl-ascorbyl-phosphate (vendu par la Sté Senju Pharmaceutical sous la référence SEPIVITAL EPC), l'ascorbyl phosphate de magnésium, l'ascorbyl phosphate de sodium (vendu par la Sté Roche sous la référence Stay-C 50) et l'ascorbyl glucoside (vendu par lan société Hayashibara) ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine^{®} ; les hormones végétales telles que les auxines et les lignanes; le palmitoyle de pentapeptide lysine-thréonine-thréonine-lysine-sérine vendu notamment sous la dénomination « MATRIXYL » par la société SEDERMA : le diméthyl amino éthanol ; les extraits de rizhome de Bupleurum Chinensis, tels que ceux vendus sous les dénominations « PLEURIMINCYL », « LIPOCARE » par la société SEDERMA ; les hydrolysats de protéine de blé acylés notamment par un groupement palmitoyle, tel que celui vendu sous la dénomination « LIPACID PVB » par la société SEPPIC ; la créatine ; le coenzyme Q10 ;
- soit sur la synthèse d'élastine, tels que l'extrait de Saccharomyces Cerivisiae commercialisé par la société LSN sous la dénomination commerciale Cytovitin^{®}; et l'extrait d'algue Macrocystis pyrifera commercialisé par la société SECMA sous la dénomination commerciale Kelpadelie^{®} ; le mélibiose ; les protéines de soja ;
- soit sur la synthèse des glycosaminoglycanes, tels que le produit de fermentation du lait par lactobacillus vulgaris, commercialisé par la société BROOKS sous la dénomination commerciale Biomin yogourth^{®} ; l'extrait d'algue brune Padina pavonica commercialisé par la société ALBAN MÜLLER sous la dénomination commerciale HSP3^{®} ; et l'extrait de Saccharomyces cerevisiae disponible notamment auprès de la société SILAB sous la dénomination commerciale Firmalift^{®} ou auprès de la société LSN sous la dénomination commerciale Cytovitin^{®} ;
- soit sur la synthèse de la fibronectine, tels que l'extrait de zooplancton Salina commercialisé par la société SEPORGA sous la dénomination commerciale GP4G^{®} ; l'extrait de levure disponible notamment auprès de la société ALBAN MÜLLER sous la dénomination commerciale Drieline^{®}; et le palmitoyl pentapeptide commercialisé par la société SEDERMA sous la dénomination commerciale Matrixil^{®};
- soit sur l'inhibition des métalloprotéinases (métalloprotéinases matricielles ou MMP) telles que plus particulièrement les MMP 1, 2, 3, 9. On peut citer: les rétinoïdes et dérivés, les oligopeptides et les lipopeptides, les lipoaminoacides, l'extrait de malt commercialisé par la société COLETICA sous la dénomination commerciale Collalift^{®} ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB^{®}), de trèfle rouge, de lin, de kakkon ou de sauge ;
- soit sur l'inhibition des sérine protéases telles que l'élastase leucocytaire ou la cathepsine G. On peut citer : l'extrait peptidique de graines de légumineuse (Pisum sativum) commercialisé par la société LSN sous la dénomination commerciale Parelastyl^{®} ; les héparinoïdes ; et les pseudodipeptides tels que l'acide {2-[acétyl-(3-trifluorométhyl-phényl)-amino]-3-méthyl-butyrylamino} acétique.

Parmi les actifs stimulant la fillagrine et les kératines, on peut citer notamment l'extrait de lupin commercialisé par la société SILAB sous la dénomination commerciale Structurine^{®} ; l'extrait de bourgeons de hêtre Fagus sylvatica commercialisé par la société GATTEFOSSE sous la dénomination commerciale Gatuline^{®} ; et l'extrait de zooplancton Salina commercialisé par la société SEPORGA sous la dénomination commerciale GP4G^{®}.

De préférence, les agents stimulant la synthèse de macromolécules dermiques ou épidermiques et/ou empêchant leur dégradation sont choisis parmi les extraits de Centella asiatica, l'acide ascorbique et ses dérivés, les peptides extraits de végétaux, tels que l'hydrolysat de soja commercialisé par la société COLETICA sous la dénomination commerciale Phytokine^{®}, l'extrait de Saccharomyces Cerivisiae commercialisé par la société LSN sous la dénomination commerciale Cytovitin^{®} ; l'extrait d'algue brune Padina pavonica commercialisé par la société ALBAN MÜLLER sous la dénomination commerciale HSP3^{®} ; les rétinoïdes et dérivés ; les extraits de romarin ; l'extrait peptidique de graines de légumineuse (Pisum sativum) commercialisé par la société LSN sous la dénomination commerciale Parelastyl^{®}; l'acide {2-[acétyl-(3-trifluorométhyl-phényl)-amino]-3-méthyl-butyrylamino} acétique ; l'extrait de lupin ; et leurs mélanges.

Comme exemples préférés de composés renforçateurs et/ou réparateurs de la fonction barrière, on peut utiliser des céramides, précurseurs ou dérivés, des stimulateurs de la synthèse de céramides, des inhibiteurs de céramidases, des sphingomyélinases, des stimulateurs de sphingomyélinases, des AGE, l'acide gamma linolénique, les acides gras insaturés en omega 3 ou omega 6, insaponifiables (beurre de karité, d'avocat, de maïs..), les galactolipides, phospholipides, le squalane, squalène.

### Agent stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8^{®} ou commercialisé par la société SILAB sous la dénomination commerciale Raffermine^{®}); et les hormones végétales telles que les giberrellines et les cytokinines.
Les agents stimulant la prolifération des kératinocytes, utilisables dans la composition selon l'invention, comprennent notamment les rétinoïdes tels que le rétinol et ses esters, dont le palmitate de rétinyle ; l'adénosine ; le phloroglucinol ; les extraits de tourteaux de noix commercialisés par la société GATTEFOSSE ; et les extraits de Solanum tuberosum commercialisés par la société SEDERMA.
Les agents stimulant la différenciation des kératinocytes comprennent par exemple les minéraux tels que le calcium ; un extrait peptidique de lupin tel que celui commercialisé par la société SILAB sous la dénomination commerciale Structurine^{®}; le beta-sitosteryl sulfate de sodium tel que celui commercialisé par la société SEPORGA sous la dénomination commerciale Phytocohésine^{®}; et un extrait hydrosoluble de maïs tel que celui commercialisé par la société SOLABIA sous la dénomination commerciale Phytovityl®; un extrait peptidique de *Voandzeia substerranea* tel que celui commercialisé par la société Laboratoires Sérobiologiques sous la dénomination commerciale Filladyn LS 9397^{®} ; et les lignanes tels que le sécoisolaricirésinol.

Comme exemples préférés de composés favorisant la prolifération kératinocytaire, on peut citer l'acide capryloil salicylique, le stimoderm^{®}, helicrisum, la criste marine, le lycopène, les extraits de tomate, la lanablue^{®}, la vitoptine^{®}, l'extrait de mamaku, la structurine^{®}, la nutelline^{®}, la caobromine.

### Agent hydratant

Par "agent hydratant", on entend :
- soit un composé agissant sur la fonction barrière, en vue de maintenir l'hydratation du *stratum corneum,* ou un composé occlusif. On peut citer les céramides, les composés à base sphingoïde, les lécithines, les glycosphingolipides, les phospholipides, le cholestérol et ses dérivés, les phytostérols (stigmastérol, β-sitostérot, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du *stratum corneum,* tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides comme le produit commercialisé sous la référence Pentavitin, le miel, les alginates (notamment le produit Sobalg PH 154 commercialisé par la société Grindsted), les carbonates cycliques, l'acide N-lauroyl pyrrolidone carboxylique ou ses sels, notamment le sel de sodium commercialisé sous la référence Nalidone, et la N-α-benzoyl-L-arginine ;
- soit un composé activant les glandes sébacées tel que les dérivés stéroïdiens (dont la DHEA, ses dérivés 7-oxydés et/ou 17-alkylés et les sapogénines), le dihydrojasmonate de méthyle, et la vitamine D et ses dérivés.
   Ces composés peuvent représenter de 0,001% à 30%, et de préférence de 0,01 à 20%, du poids total de la composition selon l'invention.

### Agent dépigmentant

Les agents dépigmentants ou anti-pigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; le D-panthétéine sulfonate de calcium, l'acide ascorbique et ses dérivés, notamment le glucoside d'ascorbyle ; et les extraits de plantes, en particulier de réglisse, de mûrier, de scutellaire et de Bacopa monnieri, sans que cette liste soit limitative.

### Agent de coloration de la peau

Cet agent peut être notamment choisi parmi un agent favorisant la mélanogénèse, un agent de coloration artificielle de la peau, et leurs mélanges.

Par 'agent favorisant la mélanogénèse' selon l'invention, on entend notamment :
- un substrat de la biosynthèse de la mélanine ;
- un stimulateur de la biosynthèse de la mélanine, les activateurs biologiques de la mélanogénèse capables d'agir :
   o en stimulant la synthèse de mélanine, et/ou
   o en stimulant l'activité ou l'expression de la tyrosinase, éventuellement par augmentation du taux d'AMPc intra-cellulaire ou par activation de la protéine kinase C, et/ou
   o en stimulant le transfert des mélanosomes des mélanocytes vers les kératinocytes, par exemple en stimulant les récepteurs PAR-2.

Ces agents favorisant la mélanogénèse peuvent être présents dans la composition en quantité comprise entre 0,1 à 15 % en poids, et de préférence en quantité comprise entre 0,5 et 5 % du poids total de la composition.

Comme 'substrat de la biosynthèse de la mélanine', on peut citer par exemple :
- la L-DOPA apportée par exemple par des extraits de plante comme Vicia faba, ou Musa sativa ;
- la L-tyrosine et ses dérivés, la L- dihydrophénylalanine.

Comme 'composés stimulant la biosynthèse de mélanine' selon l'invention, on peut citer plus particulièrement les diols aliphatiques ou cycliques tels que décrits dans J. Invest. Dermatol., 1998, 110, 4, 428 (par exemple le 5-norbornèn-2,2-diméthanol ou le 3,3-diméthyl-1,2-butanediol); les peptides de points isoélectriques compris entre 6 et 11 tels que décrit dans WO9937279 (par exemple le peptide Asp-Gln-Pro-Leu-Leu-Thr-P dans lequel P est un acide aminé hydrophobe tel que le Tryptophane Trp) ; les complexes nucléopeptidiques tels que décrits dans WO9812212 (par exemple le complexe formé par : acide purique-alanine-histidine-dibromophénylalanine-NH2); les antagonistes des récepteurs adénosine-1 (par exemple la 1,3-diméthyl-8-cyclopentylxanthine ou la 1,3-düsopropyl-8-cyclopentylxanthine), ou les agonistes de récepteurs adénosine-2 (par exemple la 2-[(cyclohexyléthyl)amino]adénosine) tels que décrits dans WO9815276 ; les complexes d'ions métalliques tels que le cuivre et de peptones tels qu'un hydrolysat protéïque provenant du soja, du collagène ou de caséïne, comme décrits dans le brevet US5,698,184. Ces documents sont tous incorporés ici par référence.

Parmi les composés stimulant l'activité ou l'expression de la tyrosinase selon l'invention, on peut citer les prostaglandines telles que décrites dans la demande de brevet WO9800100; les activateurs de NO/cGMP/protéine kinase C par exemple l'isosorbidedinitrate/cGMP/protéïne kinase C (WO9811882); ou bien encore les extraits de plantes choisis parmi Caesapinia sappan (EP820761), Parameria laevigata, Piper cubeba, Sonchus arvensis, Pluchea indica L., Massoia aromatica, Alstonia scholaris, Alycia reindwartii BI., Cinnamomum sintoc, Arctostaphylos, Chenopodium, Poterium, Gautheria (EP914816). Ces documents sont tous incorporés ici par référence.

Parmi les composés stimulant l'expression de la tyrosinase par augmentation du taux d'AMPc intracellulaire, on peut citer notamment les peptides pro-opiomélanocortiques ; les alpha-MSH ou analogues d'alpha-MSH (par exemple Ac-[D]Phe-α-MSH₁₋₁₃-NH₂) ; ou les agonistes des récepteurs MC1R (US5,683,981, WO9825584), les analogues de l'AMPc tels que décrits dans Biochem. Biophys. Res. Comm., 1987, 145, 719, la Forskoline (J. Int. Med Res., 1990, 18, 8C-17C, et les bases xanthiques (par exemple la caféine ou la théophylline).

Comme composés stimulant l'expression de la tyrosinase par activation de la protéine kinase C, on peut citer les diacylglycérols tels que décrits dans J. Invest. Dermatol., 1995, 105, 5, 687, ou bien les psoralènes tels que ceux décrits dans Photodermatol. Photoimmunol. Photomed., 1997, 13, 9. Ces documents sont tous incorporés ici par référence.

Enfin des composés stimulant le transfert des mélanosomes des mélanocytes vers les kératinocytes par stimulation des récepteurs PAR-2 ou des agonistes de PAR-2 (par exemple le peptide de composition Ser-Leu-Ile-Gly-Arg-Leu) ont été décrits dans la demande de brevet WO9904752 qui est incorporé ici par référence.

On peut également citer les polyphénols catéchiques décrits dans la demande L'OREAL WO04/080380. En particulier la catéchine, épicatéchine, gallocatéchine, épigallocatéchine, leurs sels et leurs esters, sous forme monomères ou oligomères, ainsi que les extraits végétaux en contenant (ex : extraits de thé vert).

Comme 'agent de coloration artificielle de la peau', on peut citer par exemple :
- un agent autobronzant, c'est-à-dire un agent qui, appliqué sur la peau, notamment sur le visage, permet d'obtenir un effet de bronzage d'apparence plus ou moins semblable à celui qui peut résulter d'une exposition prolongée au soleil (bronzage naturel) ou sous une lampe UV ;
- un agent de coloration additionnel, c'est-à-dire tout composé ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un pigment de maquillage ;
et leurs mélanges.

Comme exemples d'agents autobronzants, on peut citer notamment :
- la dihydoxyacétone (DHA),
- l'érythrulose, et
- l'association d'un système catalytique formé de :

- sels et oxydes de manganèse et/ou de zinc, et
- hydrogénocarbonates alcalins et/ou alcalinoterreux.

Les agents autobronzants sont généralement choisis parmi les composés mono ou polycarbonylés tels que par exemple l'isatine, l'alloxane, la ninhydrine, le glycéraldéhyde, l'aldéhyde mésotartrique, la glutaraldéhyde, l'érythrulose, les dérivés de pyrazolin-4,5-diones telles que décrites dans la demande de brevet FR 2 466 492 et WO 97/35842, la dihydroxyacétone (DHA), les dérivés de 4,4-dihydroxypyrazolin-5-ones telles que décrites dans la demande de brevet EP 903 342. On utilisera de préférence la DHA.
La DHA peut être utilisée sous forme libre et/ou encapsulée par exemple dans des vésicules lipidiques telle que des liposomes, notamment décrits dans la demande WO 97/25970.
D'une manière générale, l'autobronzant est présent en une quantité allant de 0,01 à 20 % en poids, et de préférence en quantité comprise entre 0,1 et 10 % du poids total de la composition.

Comme agents de coloration additionnels, on peut citer par exemple des extraits végétaux comme par exemple les extraits de sorgho obtenus à partir de la plante entière, des tiges, des graines ou des feuilles du genre Sorghum. Les espèces préférées du Sorghum sont choisies parmi le Sorghum bicolor, le Sorghum caudatum, le Sorghum nervosum, le Sorghum durra, le Sorghum vulgare et les Sorghum en association avec du Colletotrichum Graminicola comme ceux décrits dans la demande de brevet FR0200251.

On peut encore utiliser d'autres colorants qui permettent de modifier la couleur produite par l'agent autobronzant.
Ces colorants peuvent être choisis parmi les colorants directs synthétiques ou naturels.
Ces colorants peuvent être choisis par exemple parmi les colorants rouges ou oranges du type fluorane tels que ceux décrits dans la demande de brevet FR2840806. On peut citer par exemple les colorants suivants :
- le tetrabromofluoroscéine ou éosine connue sous le nom CTFA : CI 45380 ou Red 21
- la phloxine B connue sous le nom CTFA : CI 45410 ou Red 27
- la diiodofluorescéine connue sous le nom CTFA CI 45425 ou Orange 10 ;
- la dibromofluorescéine connue sous le nom CTFA : CI 45370 ou Orange 5.
- le sel de sodium de la tetrabromofluoroscéine connue sous le nom CTFA : CI 45380 (Na salt) ou Red 22
- le sel de sodium de la phloxine B connu sous le nom CTFA : CI 45410 (Na salt) ou Red 28
- le sel de sodium de la diiodofluorescéine connu sous le nom CTFA : Cl 45425 (Na salt) ou Orange 11 ;
- l'érythrosine connu sous le nom CTFA : CI 45430 ou Acid Red 51 ;
- la phloxine connu sous le nom CTFA : CI 45405 ou Acid Red 98.
   Ces colorants peuvent être également choisis parmi les antraquinones , le caramel, le carmin, le noir de charbon, les bleus azulènes, le methoxalène, le trioxalène, le guajazulène, le chamuzulène, le rose de bengale, la cosine 10B, la cyanosine, la daphinine.
   Ces colorants peuvent être également choisis parmi les dérivés indoliques comme les monohydroxyindoles tels que décrits dans le brevet FR2651126 (ie: 4-, 5-, 6- ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole).

### Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.
Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chorogénique ; les stilbènes, en particulier le resvératrol ; les dérivés d'acides aminés soufrés, en particulier la S-carboxyméthylcystéine ; l'ergothionéine ; la N-acétylcystéine ; des chélatants comme la N,N'-bis-(3,4,5-triméthoxybenzyl)éthylènediamine ou l'un de ses sels, complexes métalliques ou esters ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F^{®}, la fraction hydrosoluble de maïs commercialisée par la société SOLABIA sous la dénomination commerciale Phytovityl^{®}, le mélange d'extrait de fumeterre et d'extrait de citron commercialisé sous la dénomination Unicotrozon C-49^{®} par la société Induchem, et le mélange d'extraits de ginseng, de pomme, de pêche, de blé et d'orge vendu par la société PROVITAL sous la dénomination commerciale Pronalen Bioprotect^{®}.
Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl^{®}.
Enfin, comme agents piégeurs de métaux lourds utilisables dans la composition selon l'invention, on peut citer en particulier les agents chélatants tels que l'EDTA, le sel pentasodique d'éthylènediamine tétraméthylène phosphonique, et la N,N'-bis-(3,4,5-triméthoxybenzyl)éthylènediamine ou l'un de ses sels, complexes métalliques ou esters ; l'acide phytique ; les dérivés de chitosan ; les extraits de thé, en particulier de thé vert ; les tannins tels que l'acide ellagique ; les acides aminés soufrés tels que la cystéine ; les extraits de Jacinthe d'eau (Eichornia crassipes) ; et la fraction hydrosoluble de maïs commercialisée par la société SOLABIA sous la dénomination commerciale Phytovityl®.
Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase et les extraits de germes de blé en contenant, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; la guanosine ; les lignanes ; et la mélatonine.
Parmi ces agents anti-radicalaires signalons les mimétiques de Superoxide dismutase extraits de courge, melon ou encore de synthèse ainsi que les composés comme l'extrait de plancton thermal de Vitreoscilla filiformis ou son LPS purifié capables d'induire les MnSOD cutanées endogènes.

### Agent dermo-relaxant

Les agents dermo-décontractants ou dermo-relaxants utilisables dans la composition selon l'invention comprennent l'alvérine et ses sels, le gluconate de manganèse, le Diazepam, l'hexapeptide Argireline R commercialisé par la société LIPOTEC, certaines amines secondaires et tertiaires carbonylées, l'adénosine, ainsi que les sapogénines et les extraits naturels, en particulier de Wild Yam, en contenant, ainsi que les extraits de Boswellia serrata.

### Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :
(1) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les polymères d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,
(3) les protéines et hydrolysats de protéines végétales, en particulier de maïs, de seigle, de froment, de sarrasin, de sésame, d'épautre, de pois, de fève, de lentille, de soja et de lupin,
(3) les silicates mixtes, notamment les phyllosilicates et en particulier les Laponites,
(4) les microparticules de cire, choisies par exemple parmi les cires de Carnauba, de Candelila ou d'Alfa,
(5) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane.

### Agents stimulant la synthèse de la matrice extra-cellulaire

En particulier, les dérivés C-glycoside tels que décrits dans WO 02/051828 répondant à la formule (I) suivante : 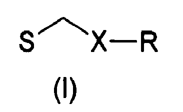 dans laquelle,
- S représente un monosaccharide ou un polysaccharide jusqu'à 20 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine éventuellement protégée,
- la liaison S-CH₂X représente une liaison de nature C-anomèrique,
- X représente un groupement choisi parmi: -CO-, -CH(NR₁R₂)-. -CHR'-, -C(=CHR')-,
- R représente une chaîne alkyle, perfluoroalkyle, hydrofluoroalkyle linéaire ou ramifiée, saturée ou insaturée, un cycle cycloalkyle, cycloperfluoroalkyle, cyclohydrofluoroalkyle, comprenant de 1 à 18 atomes de carbone, un radical phényle ou benzyle, la dite chaîne, ledit cycle ou ledit radical pouvant être éventuellement interrompu par un ou plusieurs hétéroatomes choisi parmi l'oxygène, le souffre, l'azote, le silicium, et éventuellement substituée par au moins un radical choisi parmi -OR'₁, -SR"₁, -NR'''₁R'₂, -COOR"₂, -CONHR'''₂, -CN, halogène, perfluoroalkyle, hydrofluoroalkyle et/ou au moins un radical cycloalkyle, aryle, hétérocyclique éventuellement substitués,
- R', R₁, R₂, identiques ou différents ont la même définition que celle donnée pour R, et peuvent également représenter un hydrogène et un radical hydroxyle,
- R'₁, R'₂, R''₁, R''₂, R'''₁, R'''₂, identiques ou différents, représentent un atome d'hydrogène, un radical choisi parmi un radical alkyle, hydroxyle, perfluoroalkyle et/ou hydrofluoroalkyle, linéaire ou ramifié, saturé ou insaturé, comprenant de 1 à 30 atomes de carbone.

Les agents photoprotecteurs ou filtres UVA et/ou UVB sont sous forme de composés organiques ou inorganiques, ces derniers étant éventuellement enrobés pour les rendre hydrophobes.

Comme exemples d'agents photoprotecteurs actifs dans l'UV-A et/ou l'UV-B, on peut citer désignés ci-dessus sous leur nom INCI :
- les dérivés de l'acide para-aminobenzoïique, dont les suivants : PABA, Ethyl PABA, Ethyl Dihydroxypropyl PABA, Ethylhexyl Diméthyl PABA vendu notamment sous le nom « ESCALOL 507 » par ISP, Glyceryl PABA, PEG-25 PABA vendu sous le nom « UVINUL P25 » par BASF,
- les dérivés salicyliques, dont les suivants : Homosalate vendu notamment sous le notamment sous le nom « NEO HELIOPAN OS » par Haarmann et REIMER, Dipropyleneglycol Salicylate vendu notamment sous le nom « DIPSAL » par SCHER, TEA Salicylate vendu notamment sous le nom « NEO HELIOPAN TS » par Haarmann et REIMER,
- les dérivés du dibenzoylméthane, dont les suivants : Butyl Methoxydibenzoylmethane vendu notamment sous le nom commercial « PARSOL 1789 » par HOFFMANN LAROCHE, Isopropyl Dibenzoylmethane,
- les dérivés cinnamiques, dont les suivants :Ethylhexyl Methoxycinnamate vendu notamment sous le nom commercial « PARSOL MCX » par HOFFMANN LAROCHE, Isopropyl Methoxy cinnamate, Isoamyl Methoxy cinnamate vendu notamment sous le nom commercial « NEO HELIOPAN E 1000 » par HAARMANN et REIMER, Cinoxate, DEA Methoxycinnamate, Diisopropyl Methylcinnamate, Glyceryl Ethylhexanoate Dimethoxycinnamate,
- les dérivés de β,β'-diphénylacrylate, dont les suivants : Octocrylene vendu notamment sous le nom commercial « UVINUL N539 » par BASF, Etocrylene vendu notamment sous le nom commercial « UVINUL N35 » par BASF,
- les dérivés de la benzophénone, dont les suivants : Benzophenone-1 vendue notamment sous le nom commercial « UVINUL 400 » par BASF, Benzophenone-2 vendue notamment sous le nom commercial « UVINUL D50 » par BASF, Benzophenone-3 ou Oxybenzone vendue notamment sous le nom commercial « UVINUL M40 » par BASF, Benzophenone-4 vendue notamment sous le nom commercial « UVINUL MS40 » par BASF, Benzophenone-5, Benzophenone-6 vendue notamment sous le nom commercial « Helisorb 11 » par Norquay, Benzophenone-8 vendue notamment sous le nom commercial « Spectra-Sorb UV-24 » par American Cyanamid, Benzophenone-9 vendue notamment sous le nom commercial« UVINUL DS-49» par BASF, Benzophenone-12, et le 2-(4-diéthylamino-2-hydroxybenzoyl)-benzoate de n-hexyle
- les dérivés du benzylidène camphre, dont les suivants : 3-Benzylidene camphor, 4-Methylbenzylidene camphor vendu notamment sous le nom « EUSOLEX 6300 » par MERCK , Benzylidene Camphor Sulfonic Acid, Camphor Benzalkonium Methosulfate, Terephthalylidene Dicamphor Sulfonic Acid, Polyacrylamidomethyl Benzylidene Camphor,
- les dérivés de benzimidazole, dont les suivants : Phenylbenzimidazole Sulfonic Acid vendu notamment sous le nom commercial « EUSOLEX 232 » par MERCK, Disodium Phenyl Dibenzimidazole Tetra-sulfonate vendu notamment sous le nom commercial commercial « NEO HELIOPAN AP » par Haarmann et REIMER,
- les dérivés de triazine, dont les suivants : Anisotriazine vendu notamment sous le nom commercial «TINOSORB S » par CIBA SPECIALTY CHEMICALS, Ethylhexyl triazone vendu notamment sous le nom commercial «UVINUL T150 » par BASF, Diethylhexyl Butamido Triazone vendu notamment sous le nom commercial « UVASORB HEB » par SIGMA 3V, et la 2,4,6- tris-(4'amino-benzalmalonate de diisobutyle)-s-triazine,
- les dérivés de benzotriazole, dont les suivants : Drometrizole Trisiloxane vendu sous le nom « Silatrizole » par RHODIA CHIMIE, Méthylène bis-Benzotriazolyl Tetramethylbutylphénol, vendu notamment sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,
- les dérivés anthranilique, dont le Menthyl anthranilate vendu sous le nom commercial commercial « NEO HELIOPAN MA » par Haarmann et REIMER,
- les dérivés d'imidazolines, dont l'Ethylhexyl Dimethoxybenzylidene Dioxoimidazoline Propionate,
- les dérivés de benzalmalonate, dont le polyorganosiloxane à fonctions benzalmalonate vendu sous la dénomination commerciale « PARSOL SLX » par HOFFMANN LAROCHE,
- les dérivés de 4,4-diarylbutadiène, dont le 1,1-dicarboxy (2,2'-diméthyl-propyl)-4,4-diphénylbutadiène,
- et leurs mélanges.

Les agents photoprotecteurs organiques plus particulièrement préférés sont choisis parmi les composés suivants : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de düsobutyle)-s-triazine, Anisotriazine, Ethylhexyl triazone, Diethylhexyl Butamido Triazone, Methylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

On pourra également utiliser avantageusement dans les compositions de soin et/ou de maquillage de la peau ou des lèvres selon l'invention des agents de maquillage à effet optique embellisseur de l'aspect de la peau ou des lèvres, et/ou du teint de la peau.
La quantité d'acide 8-hexadécène-1,16-dicarboxylique pourra aller de 0,00001 à 5%, de 0,00001 à 8% en poids par rapport au poids total de la composition, des quantités adaptées à la mise en oeuvre de l'invention seront notamment de 0,0001 à 0,005% ou encore 0,0001 à 0,0005% en poids par rapport au poids total de la composition.

En particulier, cet agent de maquillage à effet optique embellisseur de l'aspect et/ou du teint de la peau est choisi parmi des charges soft focus, des agents fluorescents, des azurants optiques, et leurs mélanges.

Par charge « soft-focus », on entend une charge qui en plus donne de la transparence au teint et un effet flou. De préférence, les charges «soft-focus» ont une taille moyenne des particules inférieure ou égale à 15 microns. Ces particules peuvent être de toutes formes et en particulier être sphériques ou non sphériques. De préférence encore, ces charges sont non sphériques.
Les charges « soft-focus » peuvent être choisies parmi les poudres de silice et silicates, notamment d'alumine, les poudres de type polyméthyl méthacrylate (PMMA), le talc, les composites silice/TiO₂ ou silice/oxyde de zinc, les poudres de polyéthylène, les poudres d'amidon, les poudres de polyamides, les poudres de copolymères styrène/acrylique, les élastomères de silicone, et leurs mélanges.
En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3^{®} par la société Nippon Talc, la poudre de Nylon^{®} 12, notamment celle vendue sous le dénomination Orgasol 2002 Extra D Nat Cos^{®} par la société Atochem, les particules de silice traitées en surface par une cire minérale 1 à 2 % (nom INCI : hydrated silica (and) paraffin) telles que celles commercialisées par la société Degussa, les microsphères de silice amorphe, telles que celles vendues sous la dénomination Sunsphère par exemple de référence H-53 par la société Asahi Glass, et les micro-billes de silice telles que celles vendues sous la dénomination SB-700^{®} ou SB-150^{®} par la société Miyoshi, cette liste n'étant pas limitative.
La charge à effet de flou peut être présente dans la composition cosmétique à effet de flou en une teneur allant de 0,1 à 20 % en poids et notamment allant de 1 à 12 % en poids par rapport au poids total de la composition, notamment entre 5 et 10 %, par exemple de l'ordre de 8 %.

On entend par agent fluorescent une substance qui, sous l'effet de rayons ultraviolet et/ou de la lumière visible, ré-émet dans le visible la portion de lumière qu'elle a absorbé sous la même couleur que celle qu'elle reflète naturellement. La couleur réfléchie naturellement est ainsi renforcée par la couleur ré émise et apparaît extrêmement brillante.
On peut citer par exemple les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.
On peut encore citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.
Les substances fluorescentes sont de préférence présentes dans la composition, à une teneur allant de 0,1 à 20%, de préférence de 0,1 à 15%, de préférence encore de 0,5 à 3% en poids, par rapport au poids total de la composition.

Lorsque les substances fluorescentes organiques sont blanches, on les appelle également des azurants optiques.
L'azurant optique a pour effet d'intensifier l'éclat et aviver les teintes des compositions cosmétiques les comprenant à l'application sur la peau.
Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.
De tels composés sont par exemple disponibles sous les dénominations commerciales Tinopal SOP^{®} et Uvitex OB^{®} auprès de la société CIBA GEIGY.
Les azurants optiques préférentiellement utilisés sont le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate di-sodique et/ou leurs mélanges.

Un autre objet de l'invention est l'utilisation de l'acide 8-hexadécène-1,16-dicarboxylique dans un procédé de préparation d'épidermes et/ou de peaux reconstruites, ou encore d'un modèle de follicule pileux en survie ou d'épithélium cornifié comme agent destiné à favoriser la cohésion, bonne formation, et la résistance de la couche cornée desdits épidermes et/ou des peaux reconstruites. L'étape de formation de la couche cornée est une étape délicate dans un procédé de reconstruction épidermique. Il est donc avantageux d'utiliser un composé capable de favoriser la bonne formation et la cohésion de la couche cornée dans un tel procédé.

Ces épidermes reconstruits et/ou peaux reconstruites peuvent être utilisés comme modèles pour cribler et/ou évaluer des actifs cosmétiques ou dermatologiques, ou être destinés à traiter les sujets présentant une peau lésée (grands brûlés ; excisions cutanée ; sujets atteints d'une maladie génétique affectant la peau, telle que les épidermolyses bulbeuses, *Xeroderma pigmentosum,* les ichtyoses lamellaires et les ichtyoses associées au chromosome X).
Le procédé de préparation d'épidermes reconstruits et/ou de peaux reconstruites comprend classiquement:
a) une étape de préparation d'un support ou d'un équivalent de derme; et
b) une étape d'ensemencement d'une population de kératinocytes humains sur ledit support.

L'acide 8-hexadécène-1,16-dicarboxylique pourra être additionné au milieu de culture desdites cellules, à l'une et/ou l'autre des étapes sus-décrites. La quantité d'acide 8-hexadécène-1,16-dicarboxylique présente dans le milieu de culture pourra aller de 0,001µg/ml à 20µg/ml, de préférence de 0,01µg/ml à 5µg/ml et encore plus préférentiellement de 0,1µg/ml à 1 µg/ml.

En particulier, ledit support ou équivalent de derme sera choisi parmi des lattices de collagène/ fibroblastes, un derme préalablement désépidermisé, des membranes artificielles. On peut citer à titre d'exemple de préparation de derme équivalent les protocoles décrits dans les demandes de brevets (EP-A-285471, EP-A-285474, EP-A-789074, EP-A-502172, EP-A-418035, WO-A-9116010, EP-A-197090, EP-A-20753, FR-A-2665175, FR-A-2689904) ou de préférence le protocole décrit par Asselineau et al., 1987 (Models in dermato., vol.III, Ed. Lowe & Maibach, 1-7).

On peut citer comme exemple de protocoles de préparation d'équivalents d'épiderme et/ou de peau, ceux décrits dans les brevets ou dans les demandes de brevets EP 285471, EP 285474, EP 418035, WO-A-90 02796, WO-A-9116010, EP 197090, EP 20753, FR 2665175, FR 2689904.
De manière très générale, les modèles de peau reconstruite sont constitués de kératinocytes humains déposés sur un support, souvent un équivalent de derme, et cultivés dans des conditions telles qu'ils entrent dans un programme de différenciation aboutissant à la formation d'un équivalent d'épiderme.
On peut également intégrer d'autres types cellulaires tels que les cellules de Langerhans (EP0789074) ou les mélanocytes, pour reconstituer un épiderme et/ou une peau proches des tissus natifs.

L'invention concerne également une composition de maquillage de la peau ou des lèvres comprenant, dans un milieu physiologiquement acceptable, de l'acide 8-hexadécène-1, 16-dicarboxylique en une quantité allant de 0,00001 à 3% en poids par rapport au poids total de la composition et caractérisée en ce qu'il s'agit d'une crème teintée, d'un fond de teint, d'un rouge à lèvres, d'un brillant à lèvres, ou d'un crayon de contour des lèvres, ou encore de mascara.
La quantité en acide 8-hexadécène-1,16-dicarboxylique sera de préférence supérieure ou égale à 0,0001% ; des quantités adaptées à la mise en oeuvre de l'invention seront notamment de 0,0001 à 0,005% ou encore 0,0001 à 0,0005% en poids par rapport au poids total de la composition.

L'invention se rapporte aussi à une composition cosmétique comprenant, dans un milieu physiologiquement acceptable, (a) au moins de l'acide 8-hexadécène-1,16-dicarboxylique et (b) au moins un agent de coloration de la peau.
Plus particulièrement, l'agent de coloration de la peau est choisi parmi la DHA ou les polyphénols.

L'invention se rapporte encore à une composition comprenant dans un milieu physiologiquement acceptable, (a) au moins de l'acide 8-hexadécène-1,16-dicarboxylique et (b) un autre agent favorisant l'homogénéité de la couche cornée, tel que l'urée.

La composition peut avoir la forme d'une solution aqueuse, hydroalcoolique ou huileuse éventuellement gélifiée, d'émulsion de consistance liquide ou semi-liquide du type lait, obtenue par dispersion d'une phase grasse dans une phase aqueuse (H/E) ou inversement (E/H), d'une émulsion triple (E/H/E ou H/E/H), ou de suspension ou émulsion de consistance molle, semi-solide ou solide de type crème ou gel, d'un produit anhydre liquide, pâteux ou solide ou encore de microémulsions, de microcapsules, de microparticules ou d'une dispersion vésiculaire de type ionique (liposomes ou oléosomes) et/ou non ionique (niosomes) et/ou d'une dispersion de nanosphères.

On peut également envisager une composition sous forme de mousse ou encore sous forme de spray ou d'aérosol comprenant alors un agent propulseur sous pression.

La composition peut ainsi se présenter sous forme d'une lotion, sérum, lait, crème H/E ou E/H, gel, onguent, pommade, poudre, baume, patch, tampon imbibé, savon, pain, mousse. Elle peut également se présenter sous la forme d'un stick, d'une pâte, d'une laque à lèvres ou d'une poudre, d'un fond de teint solide ou semi-solide.

De façon avantageuse, la composition de maquillage de la peau ou des lèvres pourra comprendre en outre au moins un adjuvant cosmétique choisi parmi les charges, les matières colorantes, les actifs cosmétiques hydrophiles ou lipophiles, les épaississants, les émulsionnants, les gélifiants hydrophiles ou lipophiles, les tensioactifs, les hydratants, les adoucissants, les séquestrants, les parfums, les neutralisants, les conservateurs, les antioxydants, les filtres UV, les bactéricides, les oligoéléments, les absorbeurs d'odeurs, les ajusteurs de pH et leurs mélanges.

En particulier, l'actif cosmétique pourra être notamment choisi parmi un agent stimulant la synthèse de macromolécules dermiques ou épidermiques et/ou empêchant leur dégradation, un agent stimulant la prolifération des fibroblastes ou des kératinocytes et/ou la différenciation des kératinocytes, un agent hydratant, un agent dépigmentant, un agent favorisant la coloration de la peau, un agent anti-pollution ou anti-radicalaire, un agent dermo-relaxant, un agent tenseur et leurs mélanges.
Des exemples de tels composés sont décrits précédemment.

Elle pourra comprendre en outre au moins un agent de maquillage à effet optique embellisseur de la peau et/ou des lèvres, en particulier un effet optique embellisseur du teint.
En particulier, cet agent de maquillage à effet optique embellisseur de l'aspect et/ou du teint de la peau est choisi parmi des charges soft focus, des agents fluorescents, des azurants optiques, et leurs mélanges.
Des exemples de tels composés sont décrits précédemment.

Un autre objet de l'invention est une composition cosmétique ou dermatologique comprenant, dans un milieu physiologiquement acceptable, de l'acide 8-hexadécène-1,16-dicarboxylique et au moins un acide choisi parmi l'acide lactique, l'acide glycolique, l'acide ascorbique dans un rapport quantitatif allant de 0,005 : 1 à 0,05 :1, de préférence un rapport de 0,01:1.

En particulier, la composition contient de l'acide glycolique, de l'acide 8-hexadécène-1,16-dicarboxylique et de l'acide ascorbique dans un rapport 1 : 0,01 : 1.

De préférence, l'acide 8-hexadécène-1,16-dicarboxylique est présent dans la composition en une quantité allant de 0,0001 à 0,1% en poids par rapport au poids total de la composition.

Cette composition pourra être notamment utilisée dans un procédé de peeling à effet préventif d'une altération de la formation de la couche cornée ou effet réparateur (double effet peeling et réparateur séquentiel ou simultané).
Dans une variante particulière, l'invention se rapporte à des kits comprenant une première composition comprenant au moins un agent de peeling et une seconde composition comprenant au moins de l'acide 8-hexadécène-1,16-dicarboxylique en une quantité allant de 0,00001 à 0,005% en poids par rapport au poids total de la seconde composition.
En particulier, l'agent de peeling est choisi parmi les agents kératolytiques ou les agents desquamants, les agents kératolytiques sont choisis parmi les α-hydroxy-acides ; les β-hydroxy-acides ; les α-céto-acides ; les β-céto-acides ; les rétinoïdes et les agents desquamant sont choisis parmi les β-hydroxyacides ; les α-hydroxyacides ; l'urée ; l'acide gentisique ; les oligofucoses ; l'acide cinnamique ; l'extrait de Saphora japonica ; le resvératrol et certains dérivés d'acide jasmonique ; les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

La seconde composition peut en outre comprendre un composé choisi parmi l'acide glycolique, l'acide lactique et l'acide ascorbique, plus spécifiquement tel que l'acide 8-hexadécène-1,16-dicarboxylique et l'autre acide choisi parmi l'acide lactique, l'acide glycolique, l'acide ascorbique et leurs mélanges sont présents dans la composition dans un rapport quantitatif allant de 0,005: 1 à 0,05 : 1, de préférence dans un rapport de 0,01 :1.

Enfin, cette seconde composition peut encore comprendre de l'urée dont la concentration peut être comprise entre 0,1 et 10% en poids.

Dans le procédé selon l'invention, on peut effectuer les étapes consistant à :
(i) appliquer au moins un agent desquamant à une concentration et pendant une durée induisant une activité de peeling et/ou de desquamation,
(ii) appliquer au moins l'acide 8-hexadécène-1,16-dicarboxylique ou une composition le contenant à au moins une concentration efficace pour améliorer la cohésion et/ou la formation de la couche cornée comme définies précédemment.
Les étapes (i) et (ii) pourront être simultanées ou décalées; dans ce second cas, elles pourront être interverties et/ou répétées dans le temps.

L'agent desquamant de l'étape (i) est tel que défini précédemment. Il peut notamment être choisi dans le groupe comprenant l'acide salicylique et ses dérivés, tel que l'acide n-octanoyl-5 salicylique décrit dans WO04/073605, les acides sulfoniques, les chélateurs de calcium, les α-hydroxyacides tels que les acides glycolique, citrique, lactique, tartrique, malique ou mandélique ; l'acide ascorbique et ses dérivés tels que le glucoside d'ascorbyle et l'ascorbyl phosphate de magnésium ; l'acide nicotinique, la nicotinamide; l'urée ; et l'acide (N-2 hydroxyéthylpiperazine-N-2-éthane) sulfonique (HEPES), les β-hydroxyacides tels que l'acide salicylique et ses dérivés, les rétinoïdes tels que le rétinol et ses esters, le rétinal, l'acide rétinoïque et ses dérivés, ceux décrits dans les documents FR 2 570377, EP199636, EP325540, EP402072, les extraits de châtaigne ou de figue de barbarie, en particulier commercialisés par SILAB; des composés réducteurs tels que la cystéine ou les précurseurs de cystéine.

L'invention porte encore sur un procédé cosmétique destiné à améliorer l'aspect de surface et/ou le confort de la peau, du cuir chevelu ou des lèvres, comprenant l'application topique sur la peau, le cuir chevelu ou les lèvres d'une composition de soin ou de maquillage telle que définie précédemment.

En particulier, le procédé cosmétique selon l'invention est destiné à améliorer l'homogénéité du teint et/ou favoriser la tenue d'une composition sur la peau ou les lèvres.

La composition selon l'invention peut être appliquée quotidiennement sur les lèvres ou sur l'ensemble du visage pour obtenir un teint homogène.

L'invention va être illustrée par les exemples non limitatifs suivants :

### Exemple 1 : Effet sur la formation de la couche cornée

### a) Choix des concentrations

Chaque produit à tester est appliqué pendant 24h à différentes concentrations sur des kératinocytes épidermiques humains normaux (NHEK) K015 en culture, et on évalue la dose maximale non irritante et non cytotoxique pour lesdites cellules. Cette évaluation se fait par analyse visuelle des tapis cellulaires et réduction du MTT [bromure de 3-(4,5-dimethylthiazol-2-yl)-2,5-diphenyltetrazolium], représentative de la viabilité cellulaire (Mosman T, Immunol Methods, 1983, 65: 55-63).
On obtient ainsi les doses efficaces non cytotoxiques.

Ces actifs ont ensuite été testés aux doses non irritantes et non cytotoxiques ainsi définies pour leur capacité à favoriser la bonne formation de la couche cornée.

### b) Analyse de l'expression différentielle par minichips

Afin d'étudier l'activité d'un produit vis-à-vis de la formation de la couche cornée, on analyse l'effet du produit sur l'expression de gènes connus pour être modulés dans les kératinocytes lors de leur transformation en cornéocytes.
L'analyse est réalisée à l'aide de minichips dédiées, constituées de membranes de cDNA arrays connus par l'homme de l'art pour leur relation avec la ré-épithélialisation et/ou la cohésion de la couche cornée, tels que notamment les protéines LEPs (late epidermal proteins), les cornéodesmosines, et le facteur de croissance épidermique HBEGF (human heparin-binding EGF-like growth factor).

Le modèle cellulaire utilisé comprend des kératinocytes épidermiques humains normaux (NHEK). Les kératinocytes sont cultivés en milieu SFM avec EGF 0.25ng/ml et extrait pituitaire 25µg/ml à 37°C et 5% CO₂. Les kératinocytes sont ensemencés en boites de 25cm² et pré-cultivés en milieu SFM complet, puis placés en milieu SFM sans EGF ni extrait pituitaire.

Les produits à tester sont appliqués aux concentrations non irritantes et non cytotoxiques définies en a).
Les contrôles correspondent aux mêmes conditions, en absence de produit.

Les cellules sont ensuite rincées en PBS puis lysées et l'ARN messager est extrait et purifié à l'aide du kit Chemagic mRNA Direct kit commercialisé par CHEMAGEN.
L'ARN de chaque culture est ensuite réverse-transcrit en utilisant un pool d'amorces correspondant aux ADNcs immobilisés sur les membranes (sondes) et un déoxynucléotide triphosphate marqué au ³³P.
Des séquences marquées 'cibles' ADNc multiples ont donc été réalisées pour chaque culture. Ces cibles ont ensuite été hybridées, dans des conditions optimisées, aux ADNcs 'sondes' en excès fixés sur les membranes. Après lavage, la quantité relative de cible marquée a été révélée par autoradiographie et par comptage direct sur Phosphorlmager.
L'analyse des membranes a été réalisée à l'aide du logiciel ImageQuant TL (Image analysis, Amersham Biosciences) et les données saisies ont été ensuite traitées sous Excel. On mesure ainsi l'expression différentielle des différents gènes suite au traitement en comparaison avec le témoin correspondant.

Les résultats présentés dans le tableau suivant sont exprimés par un facteur multiplicatif mettant en évidence l'activité stimulatrice des produits testés sur l'expression de gènes connus pour être impliqués dans la formation de la couche cornée.

| Actifs testés (concentration) | Marqueurs de la formation de la couche cornée (LEP) et de la cohésion intercornéocytaire (Cdsn) | Marqueurs de la ré-épithelialisation |
|---|---|---|
| Acide Lactique (100µg/ml) | LEP16 (x1.6) | - |
| Acide Glycolique (100µg/ml) | Cdsn (x1.6) | - |
| | LEP16 (x2.6) | |
| Acide Dioïque* (1 µg/ml) | Cdsn (x2.4) | HBEGF (x1.6) |
| | LEP16 (X3.0) | |
| Acide ascorbique (100µg/ml) | Cdsn (x1.7) | - |

| | | |
|---|---|---|
| *= acide 8-hexadécène-1, 16-dicarboxylique | | |

Les résultats montrent que l'acide 8-hexadécène-1,16-dicarboxylique, à faible concentration (1µg/ml), est capable de stimuler l'expression des gènes de LEP16, de la cornéodesmosine et de l'HBEGF.
Ces effets sont également obtenus pour l'acide lactique, l'acide glycolique et l'acide ascorbique à des concentrations de 100µg/ml.

Ces résultats de transcriptomique ont été confirmés par RT-PCR quantitative.

### c) RT-PCR quantitative

Les couples de primers (marqueurs) utilisés dans cette étude permettent l'amplification des fragments spécifiques suivants :
- LEP16 sous le n° GenBank NM_032563 ;
- HBEGF (human heparin-binding EGF-like growth factor) sous le n° GenBank M60278 ;
- CDSN (corneodesmosin precursor) sous le n° GenBank L20815 ;
- actine (human beta-actin) sous le n° GenBank X00351, utilisée comme contrôle.

L'ARN total restant de l'étude cDNA-array décrite en b) est utilisé. Les traces d'ADN potentiellement contaminantes sont éliminées par traitement DNAse et inactivation de DNAse. On réalise ensuite une réaction de réverse-transcription suivie d'une quantification, par fluorescence, de l'ADNc synthétisé.
Les réactions de PCR (polymerase chain reaction) ont été réalisées par PCR quantitative avec le système « Light Cycler » (Roche Molecular Systems Inc.) et selon les procédures recommandées par le fournisseur. On réalise une première série de Q-PCR sur le marqueur β-actine (contrôle) pour vérification de l'homogénéité des préparations à comparer. Puis on réalise des Q-PCR en triplicate à l'aide de couples de primers spécifiques des séquences β-actine, et des marqueurs à étudier. L'analyse de fluorescence dans l'ADN amplifié est mesurée en continu au cours des cycles de PCR. La valeur moyenne de l'expression relative (RE) est exprimée en Unités Arbitraires (UA) calculées à partir des valeurs de cycles de deux PCRs indépendantes selon la formule suivante : (1/2^{nombre de cycles}) x 10⁶.
Les résultats d'expression différentielle des 3 marqueurs LEP16, HBEGF et Cdsn sont comparés à celle de la β-actine.

L'augmentation de l'expression relative des marqueurs LEP16, HBEGF et Cdsn visualisée selon la méthode ADNc-array décrite en b) est confirmée par RT-PCR pour les produits testés.

L'ensemble de ces résultats montre que l'acide 8-hexadécène-1,16-dicarboxylique à faible concentration est capable de favoriser et/ou restaurer la formation et de la cohésion de la couche cornée.
Il est avantageux de l'utiliser dans des compositions de soin et/ou de maquillage de la peau ou des lèvres, dans des compositions capillaires de mise de forme, ainsi que pour
la préparation de compositions dermatologiques destinées au traitement des pathologies cutanées liées à une formation anormale de la couche cornée.
Et il sera encore plus avantageux de l'associer à au moins un acide choisi parmi l'acide lactique, l'acide glycolique et l'acide ascorbique pour optimiser et/ou complémenter l'effet recherché (maturation couche cornée, ré-épithélialisation).

### Exemple 2 : Formulations

| Crème de soin régénératrice | | |
|---|---|---|
| Acide 8-hexadécène-1,16-dicarboxylique | | 0,001 % |
| Acide glycolique | | 0,01% |
| Acide ascorbique | | 0,1 % |
| Méthylparaben | | 0,1% |
| Propylparaben | | 0,1% |
| Lanoline | | 5 % |
| Huile de vaseline | | 4 % |
| Huile de sésame | | 4 % |
| Alcool cétylique | | 5 % |
| Monostéarate de glycérol | | 2 % |
| Triéthanolamine | | 1 % |
| Propylène glycol | | 5 % |
| Carbomer 940 | | 0,1 % |
| Eau | qsp | 100 % |

| Fond de teint | |
|---|---|
| - Acide 8-hexadécène-1,16-dicarboxylique | 0,001 % |
| - cire microcristalline | 3% |
| - néo-pentanoate d'iso-stéaryle | 15% |
| - iso-nonanoate d'iso-nonyle | 10% |
| - cire de Carnauba | 2,% |
| - poly diméthylsiloxane (viscosité 10 CsT) | 25% |
| - oxyde de zinc | 2% |
| - oxyde de titane | 12,5% |
| - oxyde de fer jaune | 3,5% |
| - oxyde de fer noir | 0,5% |
| - conservateur | 0,2% |
| - kaolin | 3% |
| - micro-sphères de silice | 8,11% |
| - séricite (BC281 par WHITTAKER) | 10% |
| - nano- titane | 2% |

Une telle composition se présente sous la forme d'un compact anhydre coulé.

| Crème de soin de l'érythème | | |
|---|---|---|
| - Acide 8-hexadécène-1,16-dicarboxylique | | 0,004 % |
| - stéarate de glycérol | | 2,00% |
| - polysorbate 60 | | 1,00% |
| - acide stéarique | | 1,40% |
| - acide glycyrrhétinique | | 2,00% |
| - triéthanolamine | | 0,70% |
| - carbomer | | 0,40% |
| - extrait d'aloé vera | | 2,00% |
| - huile de tournesol | | 10,00% |
| - antioxydant | | 0,05% |
| - parfum | | 0,50% |
| - conservateur | | 0,30% |
| - eau | qsp | 100% |

| Composition à double effet : peeling et réparateur post-peeling | | |
|---|---|---|
| - Acide 8-hexadécène-1,16-dicarboxylique | | 0,01 % |
| - acide glycolique | | 1 % |
| - acide mandélique | | 1 % |
| - eau | | 20% |
| - polyéthylène glycol | qsp | 100% |

| Rouge à lèvres | |
|---|---|
| - Acide 8-hexadécène-1, 1 6-dicarboxylique | 0,003% |
| - Cire de polyéthylène (P.M. 500) | 12,00% |
| - Lanoline liquide | 15,00% |
| - Phényltriméthicone (DC556 de Dow Corning) | 63,34% |
| - Pigments | 8,66% |

| Lotion capillaire pour améliorer la brillance et la tenue du cheveu ainsi que l'aspect du cuir chevelu à la jonction de la racine pilaire | | |
|---|---|---|
| - Acide 8-hexadécène-1,16-dicarboxylique | | 0,1 % |
| - 2-4 DP0 | | 1.5% |
| - Valine | | 0.01% |
| - Arginine | | 0.01% |
| - éthanol | | 40% |
| - Propylène glycol | | 5% |
| - eau | QSP | 100 |

| Lotion capillaire pour favoriser la pousse du cheveu ainsi que l'aspect du cuir chevelu à la jonction de la racine pilaire | | |
|---|---|---|
| - Acide 8-hexadécène-1,16-dicarboxylique | | 0,01 % |
| - tripeptide KPV | | 0.005% |
| -Arginine | | 0.01% |
| -Taurine | | 0.01% |
| - éthanol | | 40% |
| - Propylène glycol | | 6% |
| - eau | QSP | 100 |

| | | |
|---|---|---|
| Lotion pour améliorer l'aspect du contour des ongles | | |
| - Acide 8-hexadécène-1,16-dicarboxylique | | 0,5 % |
| - Arginine | | 0.01% |
| - Ceramide-R | | 0.001% |
| - cystine B6 | | 0.0001% |
| -Taurine | | 0.001% |
| - propylène glycol | | 10% |
| - Ethanol | | 20% |
| - eau | qsp | 100 |

## Revendications

1. Utilisation non thérapeutique de l'acide 8-hexadécène-1,16-dicarboxylique dans ou pour la préparation d'une composition, comme agent destiné à favoriser la cohésion et/ou l'organisation de la couche cornée, **caractérisée en ce que** la quantité d'acide 8-hexadécène-1,16-dicarboxylique disponible est inférieure ou égale à 8% en poids par rapport au poids total de la composition.

2. Utilisation selon la revendication 1, **caractérisée en ce que** l'acide 8-hexadécène-1,16-dicarboxylique est présent à une concentration allant de 0,00001 à moins de 0,005% en poids par rapport au poids total de la composition.

3. Utilisation selon la revendication 1 ou 2, **caractérisée en ce que** l'acide 8-hexadécène-1,16-dicarboxylique est destiné à favoriser l'homogénéité et/ou la clarté du teint.

4. Utilisation selon la revendication 1 ou 2, **caractérisée en ce que** l'acide 8-hexadécène-1,16-dicarboxylique est destiné à favoriser l'homogénéité et/ou la tenue d'une composition de maquillage appliquée sur la peau ou les lèvres.

5. Utilisation selon I revendication 1 ou 2, **caractérisée en ce que** l'acide 8-hexadécène-1,16-dicarboxylique est destiné à favoriser la ré-épithélialisation et/ou la régénération de la peau ou des lèvres et du contour de la racine pilaire ou des ongles.

6. Utilisation selon la revendication 1 ou 2, **caractérisée en ce que** ledit acide 8-hexadécène-1,16-dicarboxylique est destiné à améliorer le confort la peau, du cuir chevelu, des ongles ou des lèvres, altéré par au moins une condition choisie parmi le froid, les rayonnements UV, ou des frottements mécaniques.

7. Utilisation non thérapeutique de l'acide 8-hexadécène-1,16-dicarboxylique dans une composition, pour uniformiser la couleur du bronzage.

8. Utilisation non thérapeutique de l'acide 8-hexadécène-1,16-dicarboxylique dans une composition, comme agent destiné à prévenir une altération de la cohésion de la couche cornée induite par l'action d'un actif cosmétique ou dermatologique présent dans ladite composition, et/ou favoriser sa restauration.

9. Utilisation selon la revendication 8, **caractérisée en ce que** l'actif cosmétique ou dermatologique est choisi parmi le groupe des agents kératolytiques ou desquamants, des antiséborrhéiques et/ou des anti-acnéiques, des comédolytiques, et leurs mélanges.

10. Utilisation de l'acide 8-hexadécène-1,16-dicarboxylique pour la préparation d'une composition dermatologique destinée à traiter les affections cutanées liées à une maturation anormale de la couche cornée, telles que les hyperkératoses, les parakératoses, les leukokératoses, et les troubles trophiques cutanés.

11. Utilisation selon l'une des revendication 7 à 10, **caractérisée en ce que** l'acide 8-hexadécène-1,16-dicarboxylique est présent dans la composition en une quantité allant de 0,00001 à moins de 0,005% en poids par rapport au poids total de la composition.

12. Utilisation selon l'une quelconque des revendications 1 à 11, **caractérisée en ce que** ledit acide 8-hexadécène-1,16-dicarboxylique est associé dans la composition à au moins un autre acide choisi parmi l'acide lactique, l'acide glycolique, l'acide ascorbique et leurs mélanges.

13. Utilisation selon la revendication 12, **caractérisée en ce** l'acide 8-hexadécène-1,16-dicarboxylique et l'autre acide choisi parmi l'acide lactique, l'acide glycolique, l'acide ascorbique et leurs mélanges sont présents dans la composition dans un rapport quantitatif allant de 0,005 : 1 à 0,05 :1, de préférence dans un rapport de 0,01 :1.

14. Utilisation d'acide 8-hexadécène-1,16-dicarboxylique selon l'une quelconque des revendications précédentes **caractérisée en ce que** la composition contient en outre de l'urée.

15. Utilisation selon l'une quelconque des revendications précédentes, **caractérisée en ce que** la composition contient une association d'acide 8-hexadécène-1,16-dicarboxylique, d'acide ascorbique et d'urée.

16. Procédé cosmétique d'uniformisation de la couleur du bronzage comprenant l'application sur la peau de l'acide 8-hexadécène-1,16-dicarboxylique avant ou après l'exposition au soleil.

17. Procédé selon la revendication 16, **caractérisé en ce que** la quantité d'acide 8-hexadécène-1,16-dicarboxylique va de 0,00001 à moins de 0,005%.

18. Kit comprenant une première composition comprenant au moins un agent de peeling et une seconde composition comprenant au moins de l'acide 8-hexadécène-1,16-dicarboxylique en une quantité allant de 0,00001 à 0,005% en poids par rapport au poids total de la seconde composition.

19. Kit selon la revendication 18, **caractérisé en ce que** l'agent de peeling est choisi parmi les agents kératolytiques ou les agents desquamants.

20. Kit selon la revendication 19, **caractérisé en ce que** les agents kératolytiques sont choisis parmi les α-hydroxy-acides ; les β-hydroxy-acides ; les α-céto-acides ; les β-céto-acides ; les rétinoïdes et les agents desquamant sont choisis parmi les β-hydroxyacides ; les α-hydroxyacides ; l'urée ; l'acide gentisique ; les oligofucoses ; l'acide cinnamique ; l'extrait de Saphora japonica ; le resvératrol et certains dérivés d'acide jasmonique ; les agents chélatant des sels minéraux : l'EDTA ; l'acide N-acyl-N,N',N' éthylène diaminetriacétique ; les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

21. Kit selon la revendication 18 ou 19, **caractérisé en ce que** la seconde composition comprend en outre un composé choisi parmi l'acide glycolique, l'acide lactique et l'acide ascorbique.

22. Kit selon la revendication 20, **caractérisé en ce que** l'acide 8-hexadécène-1,16-dicarboxylique et l'autre acide choisi parmi l'acide lactique, l'acide glycolique, l'acide ascorbique et leurs mélanges sont présents dans la composition dans un rapport quantitatif allant de 0,005 : 1 à 0,05 :1, de préférence dans un rapport de 0,01 :1.

23. Kit selon l'une quelconque des revendications 18 à 21, **caractérisé en ce que** la seconde composition comprend en outre de l'urée.

24. Procédé cosmétique de régénération et d'amélioration de la cohésion d'une couche cornée comprenant l'application sur la peau de l'acide 8-hexadécène-1,16-dicarboxylique après un peeling.

25. Composition de maquillage de la peau ou des lèvres comprenant, dans un milieu physiologiquement acceptable, de l'acide 8-hexadécène-1,16-dicarboxylique en une quantité allant de 0,00001 à moins de 3% en poids par rapport au poids total de la composition et **caractérisée en ce qu'**il s'agit d'une crème teintée, d'un fond de teint, d'un rouge à lèvres, d'un brillant à lèvres, ou d'un crayon de contour des lèvres.

26. Composition selon la revendication 25, **caractérisée en ce qu'**elle comprend en outre au moins un agent de maquillage choisi parmi des charges soft focus, des agents fluorescents, des azurants optiques, et leurs mélanges.

27. Composition cosmétique ou dermatologique comprenant, dans un milieu physiologiquement acceptable, (a) au moins de l'acide 8-hexadécène-1,16-dicarboxylique et (b) au moins un acide choisi parmi l'acide lactique, l'acide glycolique, l'acide ascorbique dans un rapport quantitatif allant de 0,005 : 1 à 0,05 :1, de préférence un rapport de 0,01 :1, pour une utilisation simultanée, séparée, ou décalée dans le temps.

28. Composition selon la revendication 26, **caractérisée en ce que** la composition contient de l'acide glycolique, de l'acide 8-hexadécène-1,16-dicarboxylique et de l'acide ascorbique dans un rapport 1 : 0,01 : 1.

29. Composition selon la revendication 27 ou 28, **caractérisée en ce que** l'acide 8-hexadécène-1,16-dicarboxylique est présent dans la composition en une quantité allant de 0,00001 à moins de 0,005% en poids par rapport au poids total de la composition.

30. Composition cosmétique comprenant, dans un milieu physiologiquement acceptable, (a) au moins de l'acide 8-hexadécène-1,16-dicarboxylique et (b) au moins un agent de coloration de la peau.

31. Composition selon la revendication 30, **caractérisée en ce que** l'agent de coloration de la peau est choisi parmi DHA et les polyphénols.

32. Composition comprenant dans un milieu physiologiquement acceptable, (a) au moins de l'acide 8-hexadécène-1,16-dicarboxylique et (b) un autre agent favorisant la cohésion de la couche cornée.

33. Composition selon la revendication 32, **caractérisée en ce que** ledit agent est l'urée.

34. Procédé cosmétique destiné à améliorer l'aspect de surface et/ou le confort de la peau, du cuir chevelu ou des lèvres, **caractérisé en ce que** l'on applique sur la peau, le cuir chevelu ou les lèvres une composition telle que définie dans l'une quelconque des revendications 25 à 29 ou 32 ou 33.

35. Procédé cosmétique destiné à améliorer l'homogénéité du teint et/ou favoriser la tenue d'une composition sur la peau ou les lèvres, **caractérisé en ce que** l'on applique sur la peau, le cuir chevelu ou les lèvres une composition telle que définie dans l'une quelconque des revendications 25 à 33.

36. Utilisation de l'acide 8-hexadécène-1,16-dicarboxylique dans un procédé de préparation d'épidermes et/ou de peaux reconstruites et/ou de follicules pileux en survie comme agent destiné à favoriser la cohésion et la maturation de la couche cornée desdits épidermes et/ou peaux reconstruites et /ou desdits follicules pileux en survie.
